# EUROPÄISCHE PATENTSCHRIFT

(11) **EP 1 900 294 B1**
(45) Veröffentlichungstag und Bekanntmachung des Hinweises auf die Patenterteilung: **29.06.2016**
(21) Anmeldenummer: 07017999.9
(22) Anmeldetag: 13.09.2007
(51) Int. Cl.: A23L 29/00, A23L 29/256, A23L 29/262, A23L 29/269, A23L 29/294, A23L 33/10, A23L 33/15, A23L 33/16, A23K 20/10, A23K 20/163, A23K 20/28, A23K 20/22, A23K 20/24, A23K 20/20, A23K 20/174

(54) **GELARTIGES BASENPRÄPARAT ZUR SUPPLEMENTIERUNG VON SPURENELEMENTEN UND VERFAHREN ZU DESSEN HERSTELLUNG**
GEL-LIKE BASE PREPARATION FOR SUPPLEMENTING TRACE ELEMENTS AND METHOD FOR ITS MANUFACTURE
PRÉPARATION DE BASE DE TYPE GEL POUR SUPPLÉMENTER DES OLIGOÉLÉMENTS ET SON PROCÉDÉ DE FABRICATION

(30) Priorität: 14.09.2006 DE 102006044448
(43) Veröffentlichungstag der Anmeldung: 19.03.2008
(73) Patentinhaber: Helms, Dr., Manfred, 58791 Werdohl (DE)
(72) Erfinder: Helms, Dr., Manfred, 58791 Werdohl (DE)
(74) Vertreter: Teipel, Stephan

(56) Entgegenhaltungen:
- EP-A- 0 626 175
- EP-A- 1 046 347
- EP-A1- 1 911 464
- WO-A-00/00043
- WO-A-00/36930
- WO-A-2006/108283
- DE-A1-102004 063 612
- DE-U1- 20 220 559
- FR-A- 2 796 248

## Beschreibung

Die vorliegende Erfindung betrifft ein gelartiges Basenpräparat, ein Verfahren zu dessen Herstellung und dessen Verwendung zur Supplementierung von Spurenelementen sowie zur Entsäuerung des menschlichen und tierischen Körpers.

Die meisten chronischen Erkrankungen und viele der typischen Zivilisationskrankheiten gehen einher mit einer systemischen Azidose, auch als Übersäuerung bezeichnet, die häufig auf jahrelange Ernährungsfehler zurückzuführen ist. Zu deren Prävention ist eine systematische Entsäuerung und Entgiftung des Körpers als Basisanwendung angezeigt, selbst dann, wenn noch kein spezifisches Krankheitsbild vorliegt.

Präparate zur Supplementierung von Mineralstoffen sind in verschiedenster Form am Markt erhältlich, unter anderem auch als sogenannte Nahrungsergänzungsmittel (Food-Supplements) zu Vorbeugung und/oder zur Behebung von Mangelerscheinungen und Krankheiten.

DE 10 2004 003 612 offenbart Gelbildner zur Herstellung salzhaltiger, gelartiger alkalischer Basenpräparat, die jedoch keine organische Säure enthalten.

Die WO 09/00043 offenbart ein auf Sojaprotein basierendes Nahrungsmittelprodukt, das einen pH-Wert im Bereich von 6,6 bis 7,2 aufweisen soll.

Die EP-A-1 046 347 offenbart gelartige Nahrungsmittel, die einen pH-Wert im Bereich von 3,3 - 4 aufweisen.

Die WO 2006/108283 offenbart ein Nahrungsergänzungsmittel, das neben Proteinen Fructose und Xylitol enthält.

DE 101 15 070 A1 und EP 1 245 162 A1 offenbaren ein Mittel zur Supplementierung, bestehend aus einer basischen und einer sauren Komponente. Die basische Komponente wird durch ein Carbonat und/oder Phosphat sowie ein Hydroxid oder Hydroxidcarbonat wenigstens eines Elementes aus der Gruppe Calcium, Magnesium, Natrium, Kalium, Barium und/oder Zink gebildet. Die saure Komponente enthält wenigstens eines der Spurenelemente aus der Gruppe Eisen, Kupfer, Mangan, Chrom, Molybdän und/oder Vanadium sowie wenigstens eine Säure. Der pH-Wert der sauren Komponente wird auf einen Wert unter 5 eingestellt. Es hat sich jedoch gezeigt, dass die beschriebenen Mittel zur Supplementierung eine unzureichende Wirksamkeit und Verträglichkeit aufweisen. Insbesondere kommt es sowohl bei der Ersteinnahme, als auch bei längerer Einnahme über Wochen und Monate, selbst bei einer Verabreichung in Verbindung mit Fruchtsäften, zu Übelkeit und Erbrechen bei einer Vielzahl von Anwendern.

Es besteht somit ein Bedarf an Mitteln zur Supplementierung von Spurenelementen, die die Probleme des Standes der Technik nicht aufweisen und die insbesondere ein wirksames und verträgliches Supplementieren der Spurenelemente ermöglichen.

Überraschenderweise wurde gefunden, dass ein Mittel, basierend auf einem gelartigen Basenpräparat, welchem verschiedene Spurenelemente zugefügt wurden, diese Aufgabe erfüllt.

Die vorliegende Erfindung betrifft daher ein gelartiges Basenpräparat, das eine organische Säure, eine basische Komponente, bis zu drei Gewichtsprozent eines Gelbildners, der Kieselsäure und/oder mindestens zwei Substanzen ausgewählt aus Agar Agar, Carrageen, Johannesbrotkernmehl, Guarmehl, Stärke, Stärkederivate, Cellulose, Cellulosederivate, Alginsäure, Alginate und Xanthan Gum umfasst und mindestens ein Spurenelementsalz umfasst, und das einen pH-Wert von über 8 aufweist.

Der Erfindung liegt generell die Erkenntnis zu Grunde, dass dann, wenn ein basisches Präparat Hydroxide enthält, eine praktische Darstellung in Pulverform nicht möglich, zumindest aber äußers problematisch ist. Da Hydroxide eine ausgeprägt hygroskopische Wirkung aufweisen, nehmen sie bereits bei der Verarbeitung Wasser aus der Luft auf, so dass es nicht oder nur unter Verwendung weiterer Rohstoffe möglich ist, ein rieselfähiges Pulver herzustellen. Das Pulver neigt dann überdies in Folge des durch Hydroxide vermittelten hygroskopischen Effekts zur Bildung sehr fester, in Wasser nur schwer löslicher Konglomerate.

Eine flüssige Darreichungsform des Basenpräparats, d.h. als wässrige Lösung, besitzt den Nachteil, dass speziell bei der hohen Salzkonzentration sich sehr schnell schwerlösliche Kristalle und ein fester Bodensatz bilden und diese Bestandteile dann nicht oder nur sehr schwer wieder in Lösung gebracht werden können.

Durch eine stabile Gelform sind diese Nachteile grundsätzlich vermeidbar.

Das erfindungsgemäße gelartige Basenpräparat enthält einen Gelbildner, bestehend aus mindestens zwei bis vorzugsweise vier Komponenten aus der Gruppe der Polymere, die vorzugsweise natürlichen Ursprungs sind. Beispiele der verwendbaren Polymere sind Agar Agar, Carrageen, Johannesbrotkernmehl, Guarmehl, Stärke und deren Derivate, insbesondere Xanthan Gum, Alginsäure und Alginate, Cellulose und deren Derivate, insbesondere Carboxymethyl-cellulose und/oder Kieselsäure, die als anorganischer Gelbildner eingesetzt werden kann. Die Verwendung der Kieselsäure als Gelbildner ist besonders bevorzugt, da hierdurch eine Gelbildung des Basenpräparats, das einen pH-Wert über 8 aufweist, trotz des alkalischen pH-Werts gewährleistet werden kann. Dies geschieht insbesondere dadurch, dass die im neutralen eher unlöslische kolloidale Kieselsäure im alkalischen Medium teilweise löslich ist und hierdurch der Aufbau von polymeren Kieselsäurestrukturen bewirkt wird. Kolloidale Kieselsäure als anorganischer Gelbildner bildet mit Wasser weißliche Pasten und wird u.a. als Thixotropier- und Verdickungsmittel z.B. in Zahnpasten (pH-Wert 6-8) eingesetzt.

Neben der Verwendung der Kieselsäure als Gelbildner spielt sie eine vorteilhafte Rolle bei der Verwendung des Basenpräparats zur Supplementierung von Spurenelementen und zur Entsäuerung, da sie bei der Anwendung ebenfalls von den Zellen des menschlichen Körpers aufgenommen wird und hier von Nutzen ist. Zwar zählt die Kieselsäure im Allgemeinen nicht zu den essentiellen Spurenelementen, es wird ihr jedoch eine hilfreiche Wirkung bei der Neubildung von Haut, Haaren, Nägeln, Zähnen, Knochen und (Binde-)gewebe, insbesondere eine Entsäuerung im Bereich des Nervengewebes nachgesagt. Des Weiteren reguliert die Kieselsäure die Schweißabsonderung und unterstützt somit die Ausscheidungsfunktion der Haut. Unter anderem ist sie bei Nierensteinen und Nierengriess behilflich und unterstützt das Ausleiten von Eiter über die Lymphe.

Neben der bevorzugt eingesetzten Kieselsäure können als Gelbildner andere organische Polymere, insbesondere Polysaccharide, eingesetzt werden. Diese bilden strukturell unterschiedliche Gelstrukturen, die alleine meist jedoch nicht ausreichen, um bei alkalischen pH-Werten und hohen Salzkonzentrationen eine ausreichende Gelbildung sicher zu stellen, d.h. die alleinige Verwendung eines genannten Gelbildners führt aufgrund des hohen Salzgehaltes und des hohen pH-Wertes nicht zu stabilen Zusammensetzungen. Daher werden die organischen Polymere bevorzugt in Kombination mit Kieselsäure als Gelbildner eingesetzt, wobei mindestens zwei, vorzugsweise bis zu vier verschiedene Gelbildner zum Einsatz kommen.

Das erfindungsgemäße gelartige Basenpräparat weist einen alkalischen pH-Wert von über 8, bevorzugt 8,5 bis 12,5, insbesondere 9 bis 10, z.B. 9,2 bis 9,6, auf.

Des Weiteren weist das erfindungsgemäße gelartige Basenpräparat eine hohe Konzentration an gelösten oder ungelösten Salzen auf, wobei die Gesamtsalzkonzentration 3 bis 40 Gewichtsprozent, vorzugsweise 4 bis 20 Gewichtsprozent, bezogen auf die Zusammensetzung, beträgt.

Der hohe Salzgehalt bei gleichzeitig hohem pH-Wert des erfindungsgemäßen gelartigen Basenpräparats stellt hohe Anforderungen an die eingesetzten Gelbildner, da im Allgemeinen Gelstrukturen bei hohem pH-Wert und hohem Salzgehalt zerstört werden. Dies bedeutet, dass unmittelbar nach der Herstellung ein unter Umständen noch festes oder stabiles Gel mit zunehmender Lagerung an Konsistenz verliert. Die ungelösten, unter Umständen fein verteilten schwer löslichen Bestandteile, wie z. B. Mineralsalze können sich bei unzureichender Stabilität und Festigkeit des Gels absetzen. Dies führt jedoch zu einer extrem erschwerten oder nicht mehr möglichen Verwendung des Produktes. Konventionelle Gelbildner können unter den genannten Bedingungen des hohen pH-Wertes neben hohen Salzkonzentrationen entweder keine genügende Gelbildung gewährleisten, oder sie müssen in unvertretbar hohen Konzentrationen eingesetzt werden. Das Einsetzen sehr hoher Konzentrationen von Gelbildnern führt jedoch dazu, dass sich das Endprodukt nicht mehr in Wasser löst und somit ebenfalls nicht mehr verwendbar ist.

Durch die oben genannte erfindungsgemäße Kombination von mehr als zwei bis maximal vier der oben genannten Gelbildner wird eine ausreichende Gelbildung auch bei hohem pH-Wert und hohem Salzgehalt gewährleistet. Insbesondere kann ein gelartiges Basenpräparat hergestellt werden, das längere Zeit stabil ist, und insbesondere längere Zeit eine Viskosität von vorzugsweise über 10.000 mPas aufweist, z.B. über einen Zeitraum von 24 bis 36 Monaten. Hierdurch wird unter anderem erreicht, dass eine Kristallisation der gelösten Salze und/oder eine Agglomeration ungelöster, suspendierter Salze verhindert wird. Das Gel bleibt in einem Temperaturbereich von 4 - 40° C stabil, verträgt über mehrere Stunden auch Temperaturen um ca. 80°C und ermöglicht so aufgrund der Produkthomogenität selbst bei längerer Lagerung eine gleichmäßige Dosierung durch den Verbraucher.

Die Menge der einzusetzenden Gelbildner aus der Kombination der Kieselsäure mit einem organischen Polymer oder aus zwei Polymeren aus der Liste der organischen Polymere ist so anzusetzen, dass es zur Ausbildung von stabilen Gelstrukturen und Viskositäten mit ausreichender Haltbarkeit kommt. Der jeweilige prozentuale Anteil der einzelnen Gelbildner sowie die Gesamtmenge kann bei der erfindungsgemäßen, oben beschriebenen Kombination im Vergleich zum Stand der Technik erheblich reduziert werden. Somit genügt ein Anteil des Gelbildners von bis zu 3 Gewichtsprozent, bezogen auf die Gesamtzusammensetzung, vorzugsweise bis zu 1 Gewichtsprozent, zur Herstellung eines erfindungsgemäßen gelartigen Basenpräparates.

Der hohe Salzgehalt der erfindungsgemäßen Basenpräparate gewährleistet einen Schutz gegen mikrobiellen Befall, da diese Salzkonzentration in Kombination mit dem hohen pH-Wert für die meisten Bakterien, Viren und Pilze als Lebensraum ungeeignet ist.

Das erfindungsgemäße gelartige Basenpräparat enthält eine basische Komponente. Diese besteht vorzugsweise aus Hydroxiden und/oder Hydroxid-Carbonaten und/oder Carbonaten und/oder basischen Phosphaten von mindestens einem Element der Gruppe Kalium, Natrium, Calcium und/oder Magnesium. Als Naturstoffe sind insbesondere Muschelkalk oder Dolomite als basische Komponenten bevorzugt. Die basische Komponente ist in dem gelartigen Basenpräparat in einer Menge vorhanden, damit dieses einen pH-Wert von zwischen 8,5 und 12,5, vorzugsweise zwischen 9,5 und 12,5 aufweist.

Das erfindungsgemäße gelartige Basenpräparat enthält weiterhin mindestens ein Spurenelement. Die Spurenelemente werden im Allgemeinen in Form verschiedener Salze der Elemente einer ersten Gruppe bestehend aus Eisen, Zink, Kupfer, Mangan, Chrom, Jod, Fluor, Selen und Molybdän und/oder einer zweiten Gruppe bestehend aus Brom, Lithium, Bor, Cobalt, Germanium, Nickel, Vanadium, Rubidium und Zinn eingesetzt. Die im erfindungsgemäßen gelartigen Basenpräparat eingesetzten Salze der Spurenelemente sind wasserlöslich, was zu Lösungen führt, oder wasserunlöslich, was zu Suspensionen führt.

Zur Stabilisierung der Oxidationsstufe der Spurenelemente wird mindestens eine organische Säure, vorzugsweise ausgewählt aus der Gruppe bestehend aus Ameisensäure, Essigsäure, Zitronensäure, Ascorbinsäure, Benzoesäure, Weinsäure, Glucuronsäure, Zimtsäure, Milchsäure, Salizylsäure, Oxalsäure, Malonsäure, Bernsteinsäure, Glutarsäure, Adipinsäure und/oder Äpfelsäure, insbesondere Zitronensäure und Ascorbinsäure, eingesetzt. Die organische Säure bildet mit dem Spurenelement eine Komplex, wodurch die Oxidationsstufe des Spurenelements stabilisiert wird.

Die erfindungsgemäßen gelartigen Basenpräparate können des Weiteren wasserlösliche Vitamine enthalten. Als wasserlösliche Vitamine können Substanzen aus der Gruppe Vitamin B1, B2, B6, B12, C, Folsäure, Biotin, Pantothensäure und/oder Niacin eingesetzt werden. Die erfindungsgemäßen gelartigen Basenpräparate eignen sich besonders auf Grund ihres basischen Charakters zur Supplementierung der Vitamine neben der Supplementierung der Spurenelemente, da die wasserlöslichen Vitamine im Basischen bekanntermaßen stabiler sind als im Sauren.

Die erfindungsgemäßen gelartigen Basenpräparate besitzten, insbesondere durch die Verwendung der oben beschriebenen Gelbildnerkombinationen, eine gute Löslichkeit in Wasser, was insbesondere für ein markttaugliches Produkt erforderlich ist. Somit wird dem Verbraucher eine einfache Methode zur Verfügung gestellt, z.B. eine trinkfertige Lösung bzw. Suspension mittels des erfindungsgemäßen gelartigen Basenpräparats durch einfaches Verdünnen in Wasser herzustellen. So kann z.B. auf einfachem Weg durch Einrühren eines Esslöffels des Gels (ca. 10 Gramm) in 250 ml Wasser eine trinkfertige Suspension hergestellt werden.

Diese Applikation des gelartigen Basenpräparats durch Verdünnen in Wasser hat zu dem weiter den Vorteil, dass neben der Supplementierung von Spurenelementen durch das erfindungsgemäße gelartige Basenpräparat der Anwender dazu animiert wird, eine ausreichende Menge Wasser, z.B. von 250 bis 750 ml, zu sich zu nehmen. Angesichts der Tatsache, dass die meisten Menschen zu wenig trinken, ergibt sich hier bereits ein weiterer positiver Aspekt der vorliegenden Erfindung. Außerdem sorgt die basische Lösung/Supension beim Trinken für einen pH-Wert-Ausgleich im Speichel, in der Speiseröhre, im Magen und im Dünndarm. Da dieses Mittel vorzugsweise auf leeren Magen verabreicht wird, besteht keine Gefahr einer unnötigen Belastung des Magens durch Neutralisation, da im Magen außerhalb der Verdauungszeit ohnehin nur wenig Säure vorhanden ist. Daneben ist eine Neutralisation des Magens ohnehin keine Belastung des Körpers, da die Magenschleimhaut selbst einen pH-Wert von 7 aufweist. Probleme treten eher bei zu häufiger oder zu starker Produktion von Salzsäure im Magen auf, was zu Säurereflux oder Sodbrennen führt. Diese Beschwerden können somit durch die Einnahme der basischen Lösungen/Suspensionen auf der Basis des erfindungsgemäßen Basenpräparates ebenfalls erheblich gebessert werden.

Ein weiterer wesentlicher Vorteil der erfindungsgemäßen gelartigen Basenpräparate liegt in der besonders verträglichen und wirksamen Supplementierung der enthaltenen Spurenelemente. Durch den erhöhten pH-Wert kommt es neben der vorteilhaften Neutralisation, wie oben beschrieben, zu einer begünstigten Resorption der Spurenelemente im Dünndarm. Durch die Neutralisation des sauren pH-Wertes im Magen bleibt der erhöhte pH-Wert des erfindungsgemäßen gelartigen Basenpräparates bestehen. Es kommt somit zu keiner Veränderung des pH-Wertes des gelartigen Basenpräparates, wodurch unter anderem eine Veränderung der Oxidationsstufe der enthaltenen Spurenelemente vermieden wird und diese in ionischer Form zur Resorption und Verstoffwechselung geeignet vorliegen. Gerade dies unterscheidet die erfindungsgemäßen gelartigen Basenpräparate jedoch von bekannten Spurenelemente enthaltenden Präparaten, die ein Verschieben des pH-Wertes während der Einnahme nicht zu verhindern wissen. Durch die Veränderung des pH-Wertes kommt es jedoch vorwiegend zu Redoxreaktionen der enthaltenden Spurenelemente, wodurch diese in unwirksame oder der Gesundheit oder dem Wohlbefinden abträgliche Verbindungen umgewandelt werden können. Dies könnte auch einen Mechanismus für das Entstehen von Übelkeit und Erbrechen der sauren Präparate zur Supplementierung von Spurenelementen darstellen. Durch die erfindungsgemäßen gelartigen Basenpräparate wird somit auf Grund des beständig hohen pH-Wertes die Resorption der Spurenelemente im Dünndarm, die bei einem pH-Wert oberhalb von 8 stattfinden sollte, gefördert und ein wirksames und verträgliches Supplementieren der Spurenelemente sichergestellt. Dies bestätigt sich darin, dass die erfindungsgemäßen Präparate entweder neutral oder nur schwach metallisch schmecken und es bei der Verabreichung zu keinen Übelkeitserscheinungen oder zu Erbrechen mehr kommt.

Aufgrund der wirksamen und verträglichen Supplementierung von Spurenelementen ist ein weiteres bevorzugtes Anwendungsgebiet des erfindungsgemäßen gelartigen Basenpräparates neben Nahrungsergänzung und Entsäuerung die Vorbeugung und Behandlung von Nierenfunktionsstörungen, chronischen Bindegewebsazidosen, Rheuma, Arthrose, Osteoperose, Thromboseneigung, Schlafstörungen, nervösen Erschöpfungszuständen, Bluthochdruck, Depressionen, Diabetes, Leberfunktionsstörungen, Dysbiose des Darms, Mykosen, Allergien wie z.B. Pollenallergien, Unverträglichkeitsreaktionen, z.B. auf spezielle Nahrungsmittel, Astma bronchiale, Hauterkrankungen wie Neurodermitis und Psoriasis, Haarausfall oder Varicosis.

Des Weiteren können die erfindungsgemäßen gelartigen Basenpräparate neben oral auch dermal oder transdermal angewendet werden, und zwar insbesondere bei trockener Haut, Psoriasis, Neurodermitis, Windeldermatitis, Soor- und Pilzerkrankungen, chronischen, schlecht verheilenden Wunden (z.B. Ulcus cruris), diabetischen Ulcera und Erkrankungen des rheumatischen Formenkreises (z.B. als äußerliche Rheuma- und Gelenksalbe und als unterstützende Maßnahme zu einer inneren Therapie). Für dermale Anwendungen können die erfindungsgemäßen gelartigen Basenpräparate als Zusätze für Vollbäder, Teilbäder, feuchte Auflagen, Umschläge oder auch Ganzkörperwickel eingesetzt werden. Vorteilhaft ist auch der Einsatz im Kosmetikbereich z.B. als gelförmiger Bestandteil der wässrigen Phase von kosmetischen und pharmazeutischen Emulsionen, wie Salben, Cremes, Lotionen, insbesondere als Badezusatz, wie von medizinischen entsäuernden Bädern.

In einer Ausführungsform enthalten die erfindungsgemäßen gelartigen Basenpräparate neben den Spurenelementen weitere Mineralstoffe, vorzugsweise in einer ähnlichen Menge, in der Spurenelemente vorhanden sind. Spurenelemente unterscheiden sich von Mineralstoffen darin, dass Spurenelemente, wie insbesondere die oben genannten, in Konzentrationen von weniger als 50 mg pro kg Körpertrockenmasse im menschlichen Körper nachweisbar sind. Mineralstoffe hingegen sind Elemente, die in Konzentrationen von mehr als 50 mg pro kg Köpertrockenmasse im menschlichen Körper nachweisbar sind. Als Mineralstoffe sind insbesondere Calzium, Chlor, Kalium, Magnesium, Natrium und Phosphor bekannt.

Die erfindungsgemäßen gelartigen Basenpräparate, die Spurenelemente in gleichen Größenordnungen wie Mineralstoffe enthalten sind vorteihaft, da hierdurch die Resorption von beiden Substanzklassen gefördert wird. Beim überwiegenden Vorhandensein einer Substanzklasse würde die Resorption der jeweiligen anderen Substanzklasse gestört.

Für eine effektive Entsäuerung und Entgiftung ist es vorteilhaft, zuerst mit einem Gel, das lediglich Mineralstoffe enthält, zu supplementieren, und ca. 4 bis 6 Wochen später zusätzlich das erfindungsgemäße gelartige Basenpräparat zur Supplementierung von Spurenelementen einzunehmen. Hierdurch wird sichergestellt, dass mit entsprechend hohen Mengen an Mineralstoffen der Körper in einer ersten Phase zügig entsäuert und gereinigt wird, um dann mit dem gelartigen Basenpräparat zur Supplementierung von Spurenelementen in einer zweifen Phase besonders die Entgiftung über die Leber und die Unterstützung des Immunsystems zu fördern.

Ein im Stand der Technik bestehendes erhebliches Problem ist die Herstellung eines alkalischen Gemisches, das Spurenelemente enthält, da viele Spurenelemente komplexe Redoxreaktionen in verschiedenen pH-Bereichen, insbesondere im alkalischen, eingehen können. So kommt es zu Oxidation, z.B. beim Eisen, oder es bilden sich schwerlösliche oder unlösliche Hydroxide oder Carbonate, z.B. der Elemente Eisen, Zink, Kupfer, Mangan, Molybdän, usw.

Erfindungsgemäß wurde überraschenderweise gefunden, dass durch eine spezielle Reihenfolge bei der Herstellung des erfindungsgemäßen gelartigen Basenpräparates sowohl im Bezug auf die Reihenfolge der Zugabe der Inhaltsstoffe, als auch im Bezug auf den entsprechend vorliegenden pH-Wert bei der Zugabe dieses Problem gelöst werden kann und insbesondere unerwünschte Redoxreaktionen, die zur Umwandlung der Spurenelementverbindungen in unwirksame oder nicht mehr vom Körper aufnehmbare Verbindungen führt, vermieden werden kann.

Die vorliegende Erfindung betrifft somit ebenfalls ein Verfahren zur Herstellung eines erfindungsgemäßen gelartigen Basenpräparats. Das erfindungsgemäße Verfahren zur Herstellung eines erfindungsgemäßen gelartigen Basenpräparates besteht darin, eine wässrige Lösung der Substanzen zur Stabilisierung der Oxidationsstufen der Spurenelemente vorzulegen, die vorzugsweise einen pH-Wert unter 7 aufweist, in der ausgewählte Spurenelemente, insbesondere solche, die im pH-Wert unter 7 keine unerwünschten Redoxreaktionen eingehen, gegebenenfalls unter Erwärmung einzumischen. Dies sind z.B. Eisen, Zink, Mangan, Chrom, Molybdän, Cobalt, Nickel und Vanadium. Anschließend wird die basische Komponente bis zum Erreichen des gewünschten alkalischen pH-Wertes der Zusammensetzung zugegeben, gefolgt von den Spurenelementen, die im alkalischen keine unerwünschten Redoxreaktionen eingehen. Das sind z.B. Kupfer, Fluor, Jod, Selen, Brom, Lithium, Bor, Germanium, Rubidium und Zinn. In die erwärmte Mischung wird schließlich der trocken eingewogene Gelbildner bzw. das Gemisch der Gelbildner zugegeben und intensiv homogenisiert. Nach ausreichender Abkühlung können gegebenenfalls Vitamine hinzugegeben werden. Die nach dem Abkühlen erhaltene viskose Flüssigkeit kann anschließend abgefüllt werden.

Durch das erfindungsgemäße beschriebene Verfahren wird ein gelartiges Basenpräparat zur Verfügung gestellt, dessen stabile Gelstruktur in Abhängigkeit von Salzgehalt und Gelbildnerzusammensetzung zwischen 24 und 36 Monaten lagerstabil bleibt. Bevorzugt werden beim erfindungsgemäßen Verfahren als Stabilisator der Oxidationsstufe der Spurenelemente oben angebene organische Säuren eingesetzt. Die bevorzugt verwendeten basischen Mittel zur Einstellung eines alkalischen pH-Wertes, vorzugsweise von 9 bis 11, sind die oben genannten basischen Mittel, wie sie im erfindungsgemäßen gelartigen Basenpräparat eingesetzt werden.

Durch das erfindungsgemäße Verfahren kann ein erfindungsgemäßes gelartiges Basenpräparat erhalten werden, das ein sehr vielseitiges und wirksames Spurenelementpräparat darstellt, welches nahezu den gesamten Bereich der Spurenelemente beinhalten kann und somit in der Lage ist, als Supplement aber auch als Arzneimittel einem breiten Spektrum an Mangelerscheinungen entgegen zu wirken.

Die Gelform der erfindungsgemäßen Basenpräparate ermöglicht eine Heißherstellung bei einer Temperatur von 70°C bis 90°C.

Die Erfindung betrifft weiterhin die Verwendung eines Gelbildners, der mindestens zwei Substanzen aus der Gruppe der organischen Polymere wie oben angegeben enthält, zur Herstellung eines erfindungsgemäßen gelartigen Basenpräparats zur Supplementierung von Spurenelementen, das vorzugsweise eine Salzkonzentration von 3 bis 40 Gewichtsprozent, bezogen auf die Zusammensetzung, und einen pH-Wert von 8,5 bis 12,5 aufweist.

Weiterhin betrifft die Erfindung ein gelartiges Basenpräparat, das durch das erfindungsgemäße Verfahren zur Herstellung eines gelartigen Basenpräparats erhalten werden kann.

Schließlich betrifft die vorliegende Erfindung die Verwendung eines gelartigen Basenpräparates zur Supplementierung von Spurenelementen sowie zur Entsäuerung des menschlichen und tierischen Körpers, insbesondere die orale, topische, z.B. dermale oder transdermale, Verwendung. Eine dermale Supplementierung ist möglich, da Spurenelemente auch über die Haut resorbiert werden können.

Die Zusammensetzung des erfindungsgemäßen gelartigen Basenpräparates und das Verfahren zu dessen Herstellung wird nachstehend an einigen Beispielen beschrieben.

### Beispiel 1)

### Herstellung des erfindunggemäßen gelartigen Basenpräparates

Nach Herstellung einer wässrigen Lösung der organischen Säuren zur Stabilisierung der Oxidationsstufe der Spurenelemente in der Gesamtmenge an verwendetem Wasser wurden einige der Spurenelementsalze zugegeben und anschließend erwärmt. Im Anschluss wurde in die Spurenelementmischung so viel an basischer Komponente hinzu gegeben, bis ein pH-Wert von 9 bis 11 erreicht wurde. Einige Spurenelementsalze wurden erst jetzt zugegeben. Unter intensivem Mischen wurden die trocken eingewogenen und vorgemischten Gelbildner zugegeben und die Mischung auf eine Temperatur von 70 bis 90° C bei Homogenisierungsgeschwindigkeiten von 2500 bis 3500 U/min vermischt. Nach der Abkühlung der Lösung auf ca. 40° Celsius wurden die entsprechenden Vitaminpräparate zugegeben. Es wurde eine viskose Flüssigkeit erhalten, die so in Flaschen abgefüllt eine stabile Gelstruktur aufwies. In Abhängigkeit von Salzzusammensetzung und Gelbildnerzusammensetzung blieb das Gel zwischen 24 und 36 Monaten lagerstabil.

### Beispiel 2)

Beispielhafte Zusammensetzung dreier erfindungsgemäßer gelartiger Basenpräparate zur Supplementierung von Spurenelementen

| A) | | |
|---|---|---|
| **Nr.** | **Handelsbezeichnung des Bestandteils** | **Gewichtsprozent** |
| 1 | VE-Wasser | 57 |
| 2 | Organische Säuren und Spurenelementsalze, basische Komponenten und Vitamine | 40 |
| 3 | Organische und/oder anorganischer Gelbildner | 3 |

| B) | | |
|---|---|---|
| **Nr.** | **Handelsbezeichnung des Bestandteils** | **Gewichtsprozent** |
| 1 | VE-Wasser | 79 |
| 2 | Organische Säuren und Spurenelementsalze, basische Komponenten und Vitamine | 20 |
| 3 | Organische und/oder anorganischer Gelbildner | 1 |

| C) | | |
|---|---|---|
| **Nr.** | **Handelsbezeichnung des Bestandteils** | **Gewichtsprozent** |
| 1 | VE-Wasser | 96,6 |
| 2 | Organische Säuren und Spurenelementsalze, basische Komponenten und Vitamine | 3 |
| 3 | Organische und/oder anorganischer Gelbildner | 0,4 |

### Beispiel 3)

Detaillierte Zusammensetzung dreier erfindungsgemäßer gelartiger Basenpräparate zur Supplementierung von Spurenelementen (mit Bereichen für die mengenmäßigen Anteile der Inhaltsstoffe)

| A) | | |
|---|---|---|
| **Nr.** | **Handelsbezeichnung des Bestandteils** | **Gewichtsprozent** |
| 1 | VE-Wasser | 54,00 - 96,10 |
| 2 | Organische Säuren | 13,00 - 1,50 |
| 3 | Spurenelementverbindungen | 10,00 - 0,50 |
| 4 | Basische Komponenten | 17, 00 - 1,15 |
| 5 | Wasserlösliche Vitamine | 3,00 - 0,50 |
| 6 | Alginat | 0,75 - 0,10 |
| 7 | Cellulosederivate | 0,75 - 0,10 |
| 8 | Xanthan Gum | 0,75 - 0,10 |
| 9 | Kieselsäure | 0,75 - 0,10 |

| B) | | |
|---|---|---|
| **Nr.** | **Handelsbezeichnung des Bestandteils** | **Gewichtsprozent** |
| 1 | VE-Wasser | 54,75 - 96,10 |
| 2 | Organische Säuren | 20,00 - 1,00 |
| 3 | Spurenelementverbindunqen | 5,00 - 0,50 |
| 4 | Basische Komponenten | 15,00 - 1,50 |
| 5 | Wasserlösliche Vitamine | 3,00 - 0,50 |
| 6 | Alginat | 0,75 - 0,15 |
| 7 | Xanthan Gum | 0,75 - 0,15 |
| 8 | Kieselsäure | 0,75 - 0,10 |

| C) | | |
|---|---|---|
| **Nr.** | **Handelsbezeichnung des Bestandteils** | **Gewichtsprozent** |
| 1 | VE-Wasser | 55, 50 - 96,10 |
| 2 | Organische Säuren | 8,00 - 1,25 |
| 3 | Spurenelementverbindungen | 13,00 - 0,60 |
| 4 | Basische Komponenten | 19,00 - 1,15 |
| 5 | Wasserlösliche Vitamine | 3,00 - 0,50 |
| 6 | Alginat | 0,75 - 0,20 |
| 7 | Xanthan Gum | 0,75 - 0,20 |

Ein gelartiges Basenpräparat gemäß den in den vorstehenden drei Beispielen angegebenen Zusammensetzungen ist fließfähig und kann beispielsweise in Flaschen abgefüllt in den Handel gelangen. Es handelt sich um eine viskose Flüssigkeit. Die Einnahme des Verbrauchers erfolgt dadurch, dass das gelartige Basenpräparat, z.B. die Menge eines Esslöffels (10 g) in Wasser, z.B. 250 ml, eingerührt wird. Auf diese Weise kann eine trinkfertige Lösung/Suspension (nicht alle in dem Präparat enthaltenen Salze sind vollständig löslich) erhalten werden.

An Stelle einer oralen Einnahme ist auch eine dermale/transdermale Anwendung des erfindungsgemäßen gelartigen Basenpräparats möglich, die erfindungsgemäßen gelartigen Präparate können z.B. als Badezusätze verwendet werden.

## Patentansprüche

1. Gelartiges Basenpräparat, **dadurch gekennzeichnet, dass** es eine organische Säure, eine basische Komponente, bis zu drei Gewichtsprozent eines Gelbildners, der Kieselsäure und/oder mindestens zwei Substanzen ausgewählt aus Agar Agar, Carrageen, Johannesbrotkernmehl, Guarmehl, Stärke, Stärkederivate, Cellulose, Cellulosederivate, Alginsäure, Alginate und Xanthan Gum umfasst, und mindestens ein Spurenelementsalz umfasst, und dass das Basenpräparat einen pH-Wert von über 8 aufweist.

2. Gelartiges Basenpräparat gemäß Anspruch 1, **dadurch gekennzeichnet dass** die basische Komponente wenigstens ein Hydroxid und/oder Hydroxidcarbonat und/oder Carbonat und/oder basisches Phosphat wenigstens eines Elements aus der Gruppe Kalium, Natrium, Calcium und/oder Magnesium umfasst.

3. Gelartiges Basenpräparat gemäß Anspruch 1 oder 2, worin die Gesamtsalzkonzentration 3 bis 40 Gewichtsprozent beträgt.

4. Gelartiges Basenpräparat gemäß einem der vorstehenden Ansprüche, **dadurch gekennzeichnet, dass** der Anteil an Spurenelementsalzen 0,6 bis 11 Gewichtsprozent beträgt.

5. Gelartiges Basenpräparat gemäß einem der vorstehenden Ansprüche, **dadurch gekennzeichnet, dass** das Spurenelementsalz ausgewählt ist aus einem oder mehreren Salzen der Gruppe der Elemente bestehend aus Eisen, Zink, Kupfer, Mangan, Chrom, Jod, Fluor, Selen und Molybdän und/oder der Gruppe der Elemente, bestehend aus Brom, Lithium, Bor, Cobalt, Germanium, Nickel, Vanadium, Rubidium und Zinn.

6. Gelartiges Basenpräparat gemäß einem der vorigen Ansprüche, **dadurch gekennzeichnet, dass** die Gesamtsalzmenge von 4 bis 20 Gewichtsprozent beträgt.

7. Gelartiges Basenpräparat gemäß einem der vorstehenden Ansprüche, **dadurch gekennzeichnet, dass** der pH-Wert des Basenpräparats von 8,5 bis 12,5 beträgt.

8. Gelartiges Basenpräparat gemäß einem der vorstehenden Ansprüche, **dadurch gekennzeichnet, dass** als organische Säure Ascorbinsäure oder Zitronensäure eingesetzt wird.

9. Gelartiges Basenpräparat gemäß einem der vorigen Ansprüche, **dadurch gekennzeichnet, dass** es weiterhin mindestens ein wasserlösliches Vitamin enthält.

10. Verfahren zur Herstellung eines gelartigen Basenpräparates wie in Anspruch 1 definiert, **dadurch gekennzeichnet, dass** zu einer wässrigen Lösung einer organischen Säure mindestens ein Spurenelementsalz zugegeben wird, anschließend erwärmt wird, vor, während oder nach dem Erwärmen der pH-Wert der Lösung durch Zugabe einer basischen Komponente erhöht wird, gegebenenfalls weitere Spurenelementsalze zugegeben werden und anschließend bis zu drei Gewichtsprozent eines Gelbildners, der Kieselsäure und/oder mindestens zwei Substanzen ausgewählt aus Agar Agar, Carrageen, Johannesbrotkernmehl, Guarmehl, Stärke, Stärkederivate, Cellulose, Cellulosederivate, Alginsäure, Alginate und Xanthan Gum umfasst, zugegeben werden, und gegebenenfalls nach dem Abkühlen Vitamine zugegeben werden.

11. Verfahren gemäß Anspruch 10, worin als organische Säure Ascorbinsäure oder Zitronensäure eingesetzt wird.

12. Verfahren gemäß Anspruch 10 oder 11, worin die Spurenelementsalze ausgewählt sind aus einem oder mehreren Salzen der Gruppe der Elemente, bestehend aus Eisen, Zink, Kupfer, Mangan, Chrom, Jod, Fluor, Selen und Molybdän und/oder der Gruppe der Elemente, bestehend aus Brom, Lithium, Bor, Cobalt, Germanium, Nickel, Vanadium, Rubidium und Zinn.

13. Verfahren gemäß einem der Ansprüche 10 bis 12, wobei der pH-Wert durch Zugabe der basischen Komponente auf 8,5 bis 12,5 eingestellt wird.

14. Verfahren gemäß einem der Ansprüche 10 bis 13, wobei die basische Komponente ausgewählt ist aus der Gruppe der Hydroxide und/oder Hydroxidcarbonate und/oder Carbonate und/oder basischen Phosphate der Elemente Kalium, Natrium, Calcium und/oder Magnesium.

15. Verwendung eines Gelbildners, der Kieselsäure und/oder mindestens zwei Substanzen ausgewählt aus Agar Agar, Carrageen, Johannesbrotkernmehl, Guarmehl, Stärke, Stärkederivate, Cellulose, Cellulosederivate, Alginsäure, Alginate Xanthan Gum enthält, zur Herstellung eines gelartigen Basenpräparats gemäß Anspruch 1.

16. Verwendung gemäß Anspruch 15, wobei das Basenpräparat eine Gesamtsalzkonzentration von 3 bis 40 Gewichtsprozent und einen pH-Wert von 8,5 bis 12,5 aufweist.

17. Gelartiges Basenpräparat gemäß einem der Ansprüche 1 bis 9 zur Anwendung bei der Supplementierung von Spurenelementen und der Entsäuerung im menschlichen und tierischen Körper.

18. Gelartiges Basenpräparat gemäß Anspruch 17 zur Anwendung bei der oralen oder topischen Supplementierung von Spurenelementen sowie der Entsäuerung im menschlichen und tierischen Körper.

## Claims

1. A gelatinous base preparation, **characterized in that** it comprises an organic acid, a basic component, up to three percentages by weight of a gel former comprising silicic acid and/or at least two substances selected from agar, carrageen, locust bean gum, guar gum, starch, starch derivatives, cellulose, cellulose derivatives, alginic acid, alginate, and xanthan gum, and at least one trace element salt and that the base preparation has a pH value of more than 8.

2. The gelatinous base preparation according to claim 1, **characterized in that** the basic component comprises at least one hydroxide and/or hydroxide carbonate and/or carbonate and/or basic phosphate of at least one element from the group of potassium, sodium, calcium, and/or magnesium.

3. The gelatinous base preparation according to claim 1 or 2, wherein the total salt concentration is 3 to 40 percentages by weight.

4. The gelatinous base preparation according to any one of the preceding claims, **characterized in that** the proportion of the trace element salts is 0.6 to 11 percentages by weight.

5. The gelatinous base preparation according to any one of the preceding claims, **characterized in that** the trace element salt is selected from one or more salts of the group of elements consisting of iron, zinc, copper, manganese, chromium, iodine, fluorine, selenium, and molybdenum, and/or the group of elements consisting of bromine, lithium, boron, cobalt, germanium, nickel, vanadium, rubidium, and tin.

6. The gelatinous base preparation according to any one of the preceding claims, **characterized in that** the total salt amount is 4 to 20 percentages by weight.

7. The gelatinous base preparation according to any one of the preceding claims, **characterized in that** the pH value of the base preparation is 8.5 to 12.5.

8. The gelatinous base preparation according to any one of the preceding claims, **characterized in that** ascorbic acid or citric acid is employed as the organic acid.

9. The gelatinous base preparation according to any one of the preceding claims, **characterized in that** it further contains at least one water-soluble vitamin.

10. A method for preparing a gelatinous base preparation as defined in claim 1, **characterized in that** to an aqueous solution of an organic acid there is added at least one trace element salt, subsequently it is heated, before, during and after heating the pH value of the solution is increased by adding a basic component, optionally further trace element salts are added and subsequently up to three percentages by weight of a gel former comprising silicic acid and/or at least two substances selected from agar, carrageen, locust bean gum, guar gum, starch, starch derivatives, cellulose, cellulose derivatives, alginic acid, alginate, and xanthan gum, are added, and optionally after cooling vitamins are added.

11. The method according to claim 10, wherein ascorbic acid or citric acid are employed as the organic acid.

12. The method according to claim 10 or 11, wherein the trace element salts are selected from one or more salts of the group of elements consisting of iron, zinc, copper, manganese, chromium, iodine, fluorine, selenium, and molybdenum, and/or the group of elements consisting of bromine, lithium, boron, cobalt, germanium, nickel, vanadium, rubidium, and tin.

13. The method according to any one of claims 10 to 12, wherein the pH value is adjusted to 8.5 to 12.5 by adding the basic component.

14. The method according to any one of claims 10 to 13, wherein the basic component is selected from the group of hydroxides and/or hydroxide carbonates and/or carbonates and/or basic phosphates of the elements potassium, sodium, calcium, and/or magnesium.

15. Use of a gel former containing silicic acid and/or at least two substances selected from agar, carrageen, locust bean gum, guar gum, starch, starch derivatives, cellulose, cellulose derivatives, alginic acid, alginate, xanthan gum, for the preparation of a gelatinous base preparation according to claim 1.

16. Use according to claim 15, wherein the base preparation has a total salt concentration of 3 to 40 percentages by weight and a pH value of 8.5 to 12.5.

17. The gelatinous base preparation according to any one of claims 1 to 9 for application in supplementation of trace elements and deacidification in the human and animal body.

18. The gelatinous base preparation according to claim 17 for application in oral or topical supplementation of trace elements as well as deacidification in the human and animal body.

## Revendications

1. Préparation de base de type gel, **caractérisée en ce qu'**elle comprend un acide organique, un composant basique, jusqu'à 3 % en poids d'un liant sous forme de gel, qui comprend de l'acide silicique et/ou au moins deux substances, choisies parmi l'agar-agar, les carraghénanes, de la farine de graines de caroube, de la farine de guar, de l'amidon, des dérivés d'amidon, de la cellulose, des dérivés de cellulose, de l'acide alginique, des alginates et de la gomme xanthane, et au moins un sel d'oligoélément, et **en ce que** la préparation de base présente une valeur pH supérieure à 8.

2. Préparation de base de type gel selon la revendication 1, **caractérisée en ce que** le composant basique comprend au moins un hydroxyde et/ou un carbonate d'hydroxyde et/ou un carbonate et/ou un phosphate basique au moins d'un élément issu du groupe comprenant le potassium, le sodium, le calcium et/ou le magnésium.

3. Préparation de base de type gel selon la revendication 1 ou 2, dans laquelle la concentration de sel totale présente une valeur allant de 3 à 40 % en poids.

4. Préparation de base de type gel selon l'une quelconque des revendications précédentes, **caractérisée en ce que** la proportion en sels d'oligoélément présente une valeur allant de 0,6 à 11 % en poids.

5. Préparation de base de type gel selon l'une quelconque des revendications précédentes, **caractérisée en ce que** le sel d'oligoélément est choisi parmi un ou plusieurs sels du groupe des éléments constitué du fer, du zinc, du cuivre, du manganèse, du chrome, de l'iode, du fluore, du sélénium et du molybdène et/ou du groupe des éléments, constitué du brome, du lithium, du bore, du cobalt, du germanium, du nickel, du vanadium, du rubidium et de l'étain.

6. Préparation de base de type gel selon l'une quelconque des revendications précédentes, **caractérisée en ce que** la quantité de sel totale présente une valeur allant de 4 à 20 % en poids.

7. Préparation de base de type gel selon l'une quelconque des revendications précédentes, **caractérisée en ce que** la valeur pH de la préparation de base présente une valeur allant de 8,5 à 12,5.

8. Préparation de base de type gel selon l'une quelconque des revendications précédentes, **caractérisée en ce que** l'acide ascorbique ou l'acide citrique est utilisé en tant qu'acide organique.

9. Préparation de base de type gel selon l'une quelconque des revendications précédentes, **caractérisée en ce qu'**elle contient par ailleurs au moins une vitamine hydrosoluble.

10. Procédé servant à fabriquer une préparation de base de type gel telle que définie dans la revendication 1, **caractérisé en ce qu'**au moins un sel d'oligoélément est ajouté à une solution aqueuse d'un acide organique, est immédiatement après chauffé, **en ce que** la valeur pH de la solution est augmentée, avant, pendant ou après le chauffage, par l'ajout d'un composant basique, **en ce que** le cas échéant d'autres sels d'oligoélément sont ajoutés, et **en ce que** sont ajoutés immédiatement après jusqu'à 3 % en poids d'un liant sous forme de gel, qui comprend de l'acide silicique et/ou au moins deux substances choisies parmi l'agar-agar, les carraghénanes, la farine de graines de caroube, la farine de guar, l'amidon, les dérivés d'amidon, la cellulose, les dérivés de cellulose, l'acide alginique, les alginates et la gomme xanthane, et **en ce que** des vitamines sont ajoutées le cas échéant après le refroidissement.

11. Procédé selon la revendication 10, dans lequel de l'acide ascorbique ou de l'acide citrique est utilisé en tant qu'acide organique.

12. Procédé selon la revendication 10 ou 11, dans lequel les sels d'oligoélément sont choisis parmi un ou plusieurs sels du groupe des éléments, constitué du fer, du zinc, du cuivre, du manganèse, du chrome, de l'iode, du flore, du sélénium et du molybdène et/ou du groupe des éléments, constitué du brome, du lithium, du bore, du cobalt, du germanium, du nickel, du vanadium, du rubidium et de l'étain.

13. Procédé selon l'une quelconque des revendications 10 à 12, la valeur pH étant réglée par l'ajout du composant basique à une valeur allant de 8,5 à 12,5.

14. Procédé selon l'une quelconque des revendications 10 à 13, le composant basique étant choisi parmi le groupe des hydroxydes et/ou des carbonates d'hydroxyde et/ou des carbonates et/ou des phosphates basiques des éléments que le sont le potassium, le sodium, le calcium et/ou le magnésium.

15. Utilisation d'un liant sous forme de gel, qui contient de l'acide silicique et/ou au moins deux substances choisies parmi l'agar-agar, les carraghénanes, la farine de graines de caroube, la farine de guar, l'amidon, les dérivés d'amidon, la cellulose, les dérivés de cellulose, l'acide alginique, les alginates, la gomme xanthane, aux fins de la fabrication d'une préparation de base de type gel selon la revendication 1.

16. Utilisation selon la revendication 15, la préparation de base présentant une concentration de sel totale allant de 3 à 40 % en poids et une valeur pH allant de 8,5 à 12,5.

17. Préparation de base de type gel selon l'une quelconque des revendications 1 à 9 destinée à être utilisée lors de la supplémentation d'oligoéléments et lors de la désacidification du corps de l'homme et des animaux.

18. Préparation de base de type gel selon la revendication 17 destinée à être utilisée dans le cadre de la supplémentation orale ou topique d'oligoéléments ainsi que dans le cadre de la désacidification du corps de l'homme et des animaux.
